# EUROPÄISCHE PATENTSCHRIFT

(11) **EP 2 206 461 B1**
(45) Veröffentlichungstag und Bekanntmachung des Hinweises auf die Patenterteilung: **05.10.2011**
(21) Anmeldenummer: 09004712.7
(22) Anmeldetag: 01.04.2009
(51) Int. Cl.: A61B 3/135

(54) **Verfahren und Vorrichtung zur Ausleuchtung des Mikroskopierfeldes, insbesondere des Augenhintergrundes, während Betrachtung mit einem Stereo-Spaltlampenmikroskop**
Method and device for illuminating the microscope field, particularly the eye background, during observation with a stereo silt lamp microscope
Procédé et dispositif d'éclairage du champ de microscopie, notamment du fond de l'ýil, lors de l'observation à l'aide d'un microscope à lampe à fente stéréo

(30) Priorität: 12.01.2009 EP 09000285
(43) Veröffentlichungstag der Anmeldung: 14.07.2010
(73) Patentinhaber: Tietjen, Andreas, Dr., Dr., 54311 Trierweiler (DE)
(72) Erfinder: Tietjen, Andreas, Dr., Dr., 54311 Trierweiler (DE)
(74) Vertreter: Krause, Manfred

(56) Entgegenhaltungen:
- WO-A-90/13255
- WO-A-91/06025
- DE-A1-102005 032 501
- GB-A- 1 485 592
- JP-A- 5 076 498
- US-A- 3 944 342

## Beschreibung

### Stand der Technik

In der mikroskopischen Augenheilkunde werden Auflichtstereomikroskope (meist so genannte Spaltlampenmikroskope) benutzt. Diese auf Untersuchungen menschlicher Augen spezialisierten Mikroskope erzeugen zur Beleuchtung des beobachteten Auges in einer Spaltlampe ein Spaltlicht, welches im Fokus der Beobachtungsstrahlengänge des Mikroskops fokussiert ist. Sowohl das Mikroskop (die Beobachtungsstrahlengänge), als auch der Spalt (die Beleuchtung), sind unabhängig voneinander um bis zu 180 ° horizontal um den Fokus beider Systeme schwenkbar. Zur Beobachtung des Augenhintergrundes wird, weil nur so der Augenhintergrund ausgeleuchtet werden kann, die Beleuchtung durch die Spaltlampe zwischen die Beobachtungsstrahlengänge des rechten und des linken Auges geschwenkt. ***Siehe auch*** GB-A-1485 592 ***(Rodenstock Optik* G) *14. September 1977***

Standardgeräte sind zur Anfertigung von Fotografien oder Filmen nur bedingt geeignet. Zur Fotografie oder für Filmaufnahmen wird aus einem der Beobachtungsstrahlengänge (für Stereoaufnahmen zugleich aus beiden) Licht mittels eines Strahlenteilers zur Verarbeitung in einer Kamera ausgekoppelt. Dieser Aufbau erzeugt aber ein Bild, welches im Beleuchtungs-Spalt sehr hell und beidseits neben dem Spalt sehr dunkel ist. Diese Bedingungen überfordern regelmäßig die Belichtungs-Software gängiger Digitalkameras und führen fast immer zu sehr dunklen Bildern mit einem senkrechten 'weißen Strich', dem projizierten Spalt.

Einige Hersteller von Spaltlampen kompensieren das Problem teilweise durch das Anbieten einer zusätzlich anzubringenden weiteren Lichtquelle. Diese ermöglichen dann meist passable Aufnahmen der vorderen Augenabschnitte, versagen aber überwiegend bei Untersuchungen des Augenhintergrundes. Eine zusätzliche, nicht fokussierte Lichtquelle erzeugt am Augenhintergrund entweder eine nicht hinreichende Beleuchtungsstärke, oder unerwünschte, starke Reflexe an den vorderen brechenden Medien des Auges.

Außerdem wird durch eine zusätzliche Lampe der ohnehin sehr knappe Raum vor dem Patienten-Auge weiter störend eingeschränkt. Denn im Verlauf der Untersuchung müssen durch den Untersucher Sammellinsen von 78 oder 90 Dioptrien, oder ein Dreispiegelglas nach Goldmann, zusätzlich in den Strahlengang des Mikroskops zwischen Objektiv und Patientenauge gehalten werden.

Verschiedene Hersteller haben, neben den Standardgeräten, spezialisierte Augenhintergrundmikroskope mit Foto- und Filmeinrichtungen, so genannte Funduskameras, entwickelt, mit allerdings deutlich abweichendem Aufbau der optischen Komponenten. Das Beleuchtungslicht wird hier meist als ein 'Mantel' um das Beobachtungslicht geleitet. Funduskameras kosten aber deutlich mehr als (Standard-) Spaltlampen.

### Aufgabe / Ziel der Erfindung

Das Ziel dieser Erfindung war es, mit einem (insbesondere technisch) geringen Aufwand Standard-Spaltlampenmikroskope mit einer Zusatzausrüstung zu versehen, die gute bis sehr gute Foto- oder Filmaufnahmen, insbesondere des Augenhintergrundes ermöglicht. Dabei soll der Nutzer während einer Spaltlampenuntersuchung bei Bedarf einfach Fotos anfertigen können, und somit ein störender und äußerst unerwünschter Platzwechsel von Patient und Arzt zur Fotografie an ein anderes Gerät nicht nötig sein.

### Lösung der Aufgabe

Die erfindungsgemäße Aufgabe wird gelöst, indem das Licht einer Beleuchtungslampe, insbesondere das Blitz- oder Videolicht einer handelsüblichen Digitalkamera, in einem optischen Lichtleiter gesammelt und fokussiert in das Spaltlampenmikroskop geleitet, dort mit einem für Licht teil- oder undurchlässigen Spiegel in einen der beiden Strahlengänge des Mikroskopes eingekoppelt, aus dem anderen der beiden Strahlengänge ein optisches Bild des beleuchteten Augenhintergrundes mit einem für Licht teil- oder undurchlässigen Spiegel ausgekoppelt und anschließend dem Objektiveingang der Digitalkamera zugeführt und dort eingekoppelt wird. Es wird also als eine dem Fachmann nicht nahe liegende Neuheit ein Beobachtungsstrahlengang im Mikroskop völlig zweckentfremdet und dient nun zugleich (im Falle eines für Licht teildurchlässigen Spiegels) oder alternierend (im Falle eines für Licht undurchlässigen Spiegels zum Einkoppeln des Beleuchtungslichtes) als Beleuchtungsstrahlengang.

### Nähere Erläuterung der Erfindung

Während des Schauens durch ein Spaltlampen-Mikroskop wird das Patientenauge durch die Spaltlampe beleuchtet, ein optischer Weg im Mikroskop wird vom rechten, der andere vom linken Beobachterauge benutzt.

Durch eine Montage von Strahlenteilern in die Strahlengänge wird von beiden Strahlengängen anteilig Licht ausgekoppelt. Ausgekoppeltes Licht eines der Beobachtungsstrahlengänge wird, wie vom Hersteller der Mikroskope vorgesehen, in eine Kamera geleitet und kann dort als Bild oder Film aufgezeichnet werden. Die für den Fachmann nicht nahe liegende Komponente der Lösung der Aufgabe der Erfindung liegt im vollständigen Wechsel der Beleuchtung der Mikroskopie-Objekte (insbesondere des Augenhintergrundes) während der Aufnahmen: Im zweiten Lichtweg des Mikroskops wird zur Beleuchtung des Mikroskopiergutes für Foto- oder Filmaufnahmen die Lichtrichtung umgekehrt. Dieser Strahlengang wird dann im Moment des Blitzens oder während einer andauernden Beleuchtung für Filmaufnahmen nicht zur Beobachtung benutzt, sondern er wird in diesen Zeiten ausschließlich zur Beleuchtung verwendet. Die Störung eines Strahlenganges des Stereomikroskops ausschließlich während des Fotografierens oder Filmens (ohne jegliche Einbuße in der Funktionalität während der normalen Mikroskopie) ist von Seiten des Benutzers gut akzeptabel.

Die Benutzung eines Beobachtungsstrahlenganges des Stereomikroskops zur Beleuchtung des Mikroskopsichtfeldes ist technisch elegant. Das Licht wird genau an die zu fotografierende oder zu filmende Stelle geleitet und außerdem durch die vom Benutzer eingestellte Beobachtungsvergrößerung des Mikroskops (beide Strahlengänge sind bezüglich der Vergrößerung gekoppelt) automatisch auf die gewünschte Ausdehnung - das jeweilige Sichtfeld - eingestellt. Durch die Benutzung des mikroskopeigenen Vergrößerungswechslers wird die Beleuchtung also der benutzten Vergrößerung angepasst.

Das Beleuchtungslicht ist in besonderen Ausgestaltungen der Erfindung kameraeigenes Licht, entweder das Licht einer integrierten Blitzlampe, oder das Licht einer integrierten Videolampe. Blitzlicht und Bildregistrierung einer Digitalkamera sind vom Hersteller bezüglich der verwendeten Zeitdifferenz optimiert. Aber auch jede weitere geeignete Lichtquelle kann Verwendung finden.

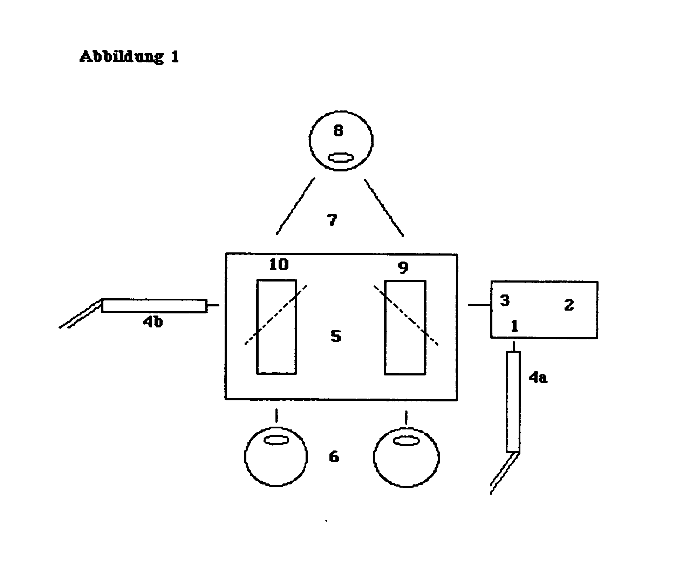

Das Beleuchtungslicht wird (über eine geeignete Einkoppeloptik) in einen Lichtleiter geleitet und aus diesem, über eine geeignete Auskoppeloptik, in den zweiten Strahlengang des Mikroskops geleitet. Der Strahlenverlauf des Beleuchtungslichtes ist dann leicht divergent. Bedingt durch diese Fokussierung der Beobachtungsstrahlengänge auf die Beobachtungsebene (vergleiche Abbildung 1) findet die Beleuchtung des Objektes in einem geringgradig anderen Einfallswinkel als die Beobachtung statt. Dadurch werden Reflexionen des Beleuchtungslichtes an optischen Grenzflächen des Patientenauges effektiv minimiert. Dabei ist es unerheblich, ob eine einfache Auflichtfotografie durchgeführt wird, oder außerhalb des Mikroskopes besondere Strahlengänge durch zusätzliche Lupen oder Kontaktgläser bei einer Fundusskopie durch den Augenarzt benutzt werden.

Die Fokussierung der Foto- oder Filmbilder erfolgt über den Sucher der Kamera. Per Lichtleiter wird das von der Kamera ausgesendete Licht an die gewünschte Stelle gebracht und das reflektierte Licht vom Kameracomputer entsprechend den Bedürfnissen der Kamera verarbeitet. Dies ermöglicht die Nutzung jeweils neuester Kameramodelle ohne kostenintensive Adaptation.

Dieses Verfahren und die zugehörigen Vorrichtungen ermöglichen keine Stereofotografie, da nur ein Strahlengang mit einer Kamera verbunden ist. Möglich ist aber eine einfache, qualitativ hochwertige, mon-okulare Fotografie zur Dokumentation des im Blickfeld Sichtbaren. Eine Stereofotografie ist aber in sehr vielen Fällen des augenärztlichen Handelns auch gar nicht nötig.

Die Vorteile der Erfindung sind, zusammengefasst:
- Die Möglichkeit der Nachrüstung gewöhnlicher Spaltlampenmikroskope,
- die Möglichkeit der Verwendung von Kameras jeweils neuester Bauart, ohne die Notwendigkeit einer elektronischen Adaptation,
- der Erhalt der Herstellergarantie und der Verkäufergewährleistung der Kamera, da diese nicht verändert wird,
- eine uneingeschränkte Nutzung der Kameralogik zur Belichtungs- und Beleuchtungssteuerung,
- eine zusätzliche Möglichkeit der Einleitung von Licht verschiedener Lichtquellen,
- die Möglichkeit einer Modifizierung des Beleuchtungslichtes durch Filter.

### Beispiele

### Beispiel 1

In einem Spaltlampen-Mikroskop SL 120, Fa. Carl Zeiss Jena, D wurden in die beiden Beobachtungstrahlengänge teildurchlässige Spiegel des Gerätezubehörs (zur Auskopplung von Bildern) in die geräteseitig vorhandenen Halterungen platziert. Der Objektiveingang einer Digitalkamera Canon FS 11, Fa. Canon, Tokio, Japan wurde mit Hilfe einer passgenauen (selbst gefertigten) Halterung vor dem Ausgang eines der durch die teildurchlässigen Spiegel ausgekoppelten Teilstrahlengänge zentriert platziert. Selbst auf Maß gefertigte Halterungen für einen optischen Lichtleiter Fa. Volpi, Schlieren, CH wurden am Blitz- und Videolichtausgang der Digitalkamera und am zweiten Ausgangsstutzen für ausgekoppeltes Licht des zweiten Strahlenganges des Mikroskopes (hier als Eingang für einzukoppelndes Beleuchtungslicht) angebracht und der Lichtleiter eingesetzt.

Entsprechend den Klassifizierungsregeln der Richtlinie 93/42/EWG über Medizinprodukte (MDD) bleibt die Spaltlampe weiterhin ein nicht invasives, aktives Medizinprodukt der Klasse 1.

Mit dieser Vorrichtung wurden von verschiedenen Freiwilligen Aufnahmen ihres Augenhintergrundes angefertigt. Die Aufnahmen waren gut ausgeleuchtet und insgesamt von guter Qualität.

### Abbildung 1:

Allgemeines Schema der erfindungsgemäßen Vorrichtung:
   (1) Beleuchtungslampe (hier als integrierte Lampe der
   (2) Digital Kamera mit deren
   (3) Objektiveingang
   (4a) Lichtleiter zur Aufnahme des Lichtes von (1) und
   (4b) Abgabe des Lichtes in den
   (5) Strahlenteiler.
   (6) Mikroskopokulare (während der Erfindungsgemäßen Nutzung ohne Funktion)
   (7) Mikroskopobjektiv
   (8) Beobachtetes Gut (Patient)
   (9) Bildlichtführung
   (10) Beleuchtungslichtführung (Erfindungsgemäß)

## Patentansprüche

1. Verfahren zur Beleuchtung von Mikroskopiergut, insbesondere eines Augenhintergrundes, während dessen Betrachtung mit einem augenärztlichen Stereo-Spaltlampenmikroskop, zur Fotografie oder Anfertigung von Filmen, wobei aus einem der beiden Beobachtungs-Strahlengänge des Spaltlampenmikroskopes ein Mikroskopbild ausgekoppelt und anschließend einer Kamera zugeführt wird,
**dadurch gekennzeichnet,**
**dass** das Licht einer Beteuchtungslampe in das Spaltlampenmikroskop geleitet und dort durch Überlagerung retrograd in den anderen der beiden optischen Beobachtungs-Strahlengänge des Mikroskopes eingekoppelt wird.

2. Verfahren gemäß Anspruch 1,
**dadurch gekennzeichnet,**
**dass** zur Fotografie als Beleuchtungslicht das Blitzlicht einer integrierten Blitzlampe der verwendeten Foto-Kamera verwendet wird.

3. Verfahren gemäß Anspruch 1,
**dadurch gekennzeichnet,**
**dass** zum Filmen als Beleuchtungslicht das Videolicht einer integrierten Videolampe der verwendeten Film-Kamera verwendet wird.

4. Vorrichtung umfassend optische Bauteile und Geräte zur Beleuchtung eines Mikroskopierfeldes, insbesondere eines Augenhintergrundes, eines augenärztlichen Stereo-Spaltlampenmikroskops bei dem aus einem *der beiden optischen Strahlengänge mittels eines optischen Spiegels ein Mikroskopbild ausgekoppelt und dem Objektiveingang* (3) *einer Kamera (4)* zur Fotografie oder zur Anfertigung von Filmen *zugeführt ist,*
**gekennzeichnet durch**
eine Beleuchtungslampe (1), deren Licht in das Spaltlarnpenmikroskop geleitet und dort durch Überlagerung mittels eines optischen Spiegels retrograd in *den anderen* der beiden optischen Strahlengänge des Mikroskopes eingekoppelt ist.

5. Vorrichtung gemäß Anspruch 4,
**dadurch gekennzeichnet,**
**dass** zum Einkoppeln des Beleuchtungslichtes und zum Auskoppeln des Mikroskopbildes für Licht teildurchlässige oder undurchlässige Spiegel vorgesehen sind.

6. Vorrichtung gemäß Anspruch 4 oder 5,
**dadurch gekennzeichnet,**
**dass** zum Leiten des Beleuchtungslichtes in das Mikroskop ein optischer Lichtleiter vorgesehen sind.

7. Vorrichtung gemäß einem der Ansprüche 4 bis 6,
**dadurch gekennzeichnet,**
**dass** zur Fotografie die Beleuchtungslampe (1) die integrierte Blitzlampe der verwendeten Fotokamera (2) ist.

8. Vorrichtung gemäß einem der Ansprüche 4 bis 6,
**dadurch gekennzeichnet,**
**dass** zum Filmen die Beleuchtungslampe (1) die integrierte Videolampe der verwendeten Filmkamera ist.

## Claims

1. To have adapted the process for illuminating a microscope's subject, in particular the ocular fundus, during an eye examination using an ophthalmological stereo slit lamp microscope in order to shoot photographs or film. One of the microscope's two optical light paths is diverted and directed into a camera, which produces an image of the microscope's subject.
The process features:
the light of an illuminating light source being directed into the slit lamp microscope, where it is fed, in the reverse direction at the same time, into the other of the microscope's two optic paths.

2. To have created the process outlined in point 1,
which features
the light from the camera's integrated flash being used as the illuminating light source.

3. To have created the process outlined in point 1,
which features
the light from the video camera's integrated light being used as the illuminating light source.

4. To have created an apparatus, comprising optical components and devices for illuminating a stereo slit lamp microscope's field of vision, in particular the ocular fundus. One of the microscope's two optical light paths is diverted via an optical mirror and directed into the objective of a camera, which produces photos or film of the microscope's subject.
The apparatus includes:
an illuminating light source (1), whose light is directed into the slit lamp microscope, where it is fed, via an optical mirror, into the other of the microscope's two optic paths, where it travels in the reverse direction at the same time.

5. To have created the apparatus outlined in point 4,
which includes
semi and fully-opaque mirrors that are used to feed in the illuminating light and to divert the light to produce an image of the microscope's subject.

6. To have created the apparatus outlined in points 4 and 5,
which includes
an optical light guide that is used to direct the illuminating light beam into the microscope.

7. To have created the apparatus outlined in points 4 to 6,
which includes
the flash (1) integrated into the camera (2) as the illuminating light source for the photographs.

8. To have created the apparatus outlined in points 4 to 6,
which includes
the light (1) integrated into the video camera as the illuminating light source for the film.

## Revendications

1. Procédé servant à l'éclairage d'objets microscopiques, notamment du fond de l'oeil, pendant l'observation de ce dernier au moyen d'un stéréomicroscope à lampe à fente, à usage ophtalmologique, à des fins photographiques ou cinématographiques ; de l'une des deux trajectoires de faisceau d'observation de la microscope à lampe fendue, une image microscopique est découplée et acheminée vers un appareil photo ou une caméra ; ce procédé est **caractérisé par le fait**
**que** la lumière d'une lampe d'éclairage est dirigée dans le microscope à lampe à fente, avant d'être couplée par superposition rétrograde à l'autre des deux trajectoires de faisceau d'observation du microscope.

2. Procédé conforme à la revendication 1,
**caractérisé par le fait**
**qu'**en prenant des photos, on utilise comme lumière d'exposition le flash d'une lampe éclair intégrée dans l'appareil photo.

3. Procédé conforme à la revendication 1,
**caractérisé par le fait**
**qu'**en filmant, on utilise comme lumière d'exposition la lumière vidéo d'une lampe vidéo intégrée à la caméra cinématographique utilisée.

4. Dispositif comprenant des composants optiques et des appareils servant à l'éclairage du champ microscopique, notamment du fond de l'oeil, d'un stéréomicroscope à lampe à fente, à usage ophtalmologique, pour lequel l'une des trajectoires de faisceau est découplée au moyen d'un miroir optique, donnant une image microscopique, laquelle est acheminée vers l'entrée de l'objectif (1) d'un appareil photo (4) ou d'une caméra servant à faire des films ; ce dispositif est **caractérisé par**
une lampe d'éclairage (1) dont la lumière est acheminée dans le microscope à lampe à fente, où elle est couplée par superposition rétrograde et via un miroir optique à l'autre des deux trajectoires à faisceaux optiques.

5. Dispositif conforme à la revendication 4,
**caractérisé par le fait que**
pour le couplage de la lumière d'éclairage et pour le découplage de l'image microscopique, des miroirs opaques ou semi-opaques sont prévus.

6. Dispositif conforme aux revendications 4 ou 5,
**caractérisé par le fait**
**qu'**un conducteur optique est prévu pour l'acheminement de la lumière d'éclairage vers le microscope.

7. Dispositif conformes à l'une des revendications 4 à 6,
**caractérisé par le fait**
**que** pour prendre des photos, la lampe-éclair intégrée de l'appareil photo (2) est utilisée comme lampe d'éclairage (1).

8. Dispositif conforme à l'une des revendications 4 à 6,
**caractérisé par le fait**
**que** pour filmer, la lampe vidéo de la caméra est utilisée comme lampe d'éclairage (1).
